# DEMANDE DE BREVET EUROPEEN

(11) **EP 0 608 154 A1**
(43) Date de publication de la demande: **27.07.1994**
(21) Numéro de dépôt: 94400016.5
(22) Date de dépôt: 04.01.1994
(51) Int. Cl.: A61L 11/00, A61L 2/08

(54) **Installation de décontamination de produits contenant des microorganismes, notamment des déchets hospitaliers**

(30) Priorité: 05.01.1993 FR 9300034
(71) Demandeur: Icre, Pierre, F-78000 Versailles (FR); COMPAGNIE FRANCAISE DE RENOVATION - COFRAR, F-78000 Versailles (FR)
(72) Inventeur: Icre, Pierre, F-78000 Versailles (FR); de Rocquigny du Fayel, Hugues, F-78000 Versailles (FR)
(74) Mandataire: Polus, Camille

(57) **Abrégé**

Cette installation comprend une enceinte (12) de traitement des produits, délimitée par des parois étanches aux rayonnements ionisants, et une source de rayonnements ionisants (44) montée à l'intérieur de l'enceinte en étant déplaçable dans une position sortie de traitement des produits et une position escamotée de stockage. Cette installation comporte de plus des moyens (82) de chauffage des produits contenus dans l'enceinte de traitement (12). L'installation comporte également un dispositif (100) d'extraction de l'atmosphère interne de l'enceinte chargée en ozone. La source de rayonnements (44) est constituée d'un radioélément artificiel tel que le cobalt 60 ou le césium 137.

## Description

La présente invention concerne une installation de décontamination de produits contenant des microorganismes, notamment des déchets hospitaliers.

Il est connu de traiter les déchets hospitaliers dans des incinérateurs installés sur le site même de l'hôpital. Cependant, les fumées rejetées par ces incinérateurs ne vérifient pas les nouvelles normes applicables en matière de pollution et ces incinérateurs sont destinés à être abandonnés.

On connaît par ailleurs dans l'état de la technique des procédés et des installations de stérilisation de produits contenant des microorganismes, tels que du matériel médico-chirurgical ou des denrées alimentaires, au moyen de rayonnements ionisants tels que le rayonnement gamma émis par exemple par une source au cobalt 60.

FR-A-2 590 717 décrit une installation de décontamination de produits contenant des microorganismes, du type comprenant une enceinte de traitement des produits, délimitée par des parois étanches aux rayonnements ionisants, et une source de rayonnements ionisants montée à l'intérieur de l'enceinte en étant déplaçable entre une position sortie de traitement des produits et une position escamotée de stockage.

La radiosensibilité ou la radiorésistance des différents microorganismes habituellement présents dans les déchets hospitaliers est parfaitement connue. Par conséquent, après évaluation de la nature et de la quantité des microorganismes contaminant un ensemble de déchets hospitaliers, il est possible de déterminer la dose de rayonnements qu'il est nécessaire de délivrer à cet ensemble pour le décontaminer.

Cependant, compte tenu de la contamination élevée de certains déchets hospitaliers dits "à risque", en particulier par des germes radiorésistants, il est nécessaire d'envisager des doses de rayonnements importantes pour garantir une décontamination satisfaisante.

L'invention a notamment pour but de décontaminer des déchets hospitaliers de manière à pouvoir les transporter ou les traiter ultérieurement au même titre que des déchets ménagers courants, ceci au moyen d'une installation simple et compacte, que l'on puisse implanter sur le site même de production des déchets, mettant en oeuvre des doses de rayonnements ionisants relativement faibles.

Acet effet l'invention a pour objet une installation de décontamination de produits contenant des microorganismes, notamment des déchets hospitaliers, du type précité, caractérisée en ce que l'enceinte comporte un tiroir de mise en place des produits à traiter monté coulissant, à travers une ouverture de l'enceinte, entre une position ouverte de chargement ou de déchargement des produits et une position fermée de traitement des pro-duits.

Suivant d'autres caractéristiques de l'invention:
- le tiroir comporte une porte d'obturation de l'ouverture de chargement, étanche aux rayonnements, et un réceptacle de chargement des produits à traiter, solidaire de la face interne de la porte ;
- l'extrémité distale du réceptacle est délimitée par une paroi formant une barrière empêchant l'accès d'un opérateur à l'intérieur de l'enceinte lorsque le tiroir est en position ouverte ;
- le réceptacle comporte un évidement central formant logement pour la source de rayonnements en position sortie de manière que les produits à traiter soient disposés autour de cette source;
- le réceptacle comporte un évidement longitudi- i-nal, s'étendant entre l'extrémité distale du réceptacle et l'évidement central, formant un passage pour un câble de manutention de la source de rayonnements lors du déplacement du tiroir ;
- le réceptacle comporte au moins un portillon pour le chargement ou le déchargement des produits à traiter ;
- la zone, adjacente à l'enceinte, dans laquelle s'effectue la course du tiroir, est clôturée par une barrière de sécurité ;
- la barrière de sécurité comporte au moins une porte d'accès à la zone clôturée, disposée de manière à être sensiblement en vis-à-vis avec le portillon du réceptacle lorsque le tiroir est en position-ouverte ouverte ;
- le dessus de l'enceinte comporte un orifice de passage d'un organe de manutention d'un conteneur de stockage de la source de rayonnements, l'orifice de passage étant obturé par un bouchon amovible destiné à être retenu en position d'obturation par accouplement avec un organe de sécurité, fixé à l'intérieur de l'enceinte, formant support pour une poulie de renvoi d'un câble de manutention de la source de rayonnements ;
- l'organe de sécurité comporte une plaque fermant l'extrémité de l'orifice de passage débouchant dans l'enceinte ; ;
- l'installation comprend des moyens de chauffage des produits disposés dans l'enceinte de traitement ;
- les moyens de chauffage comprennent un dispositif de soufflage d'air chaud dans l'enceinte;
- le dispositif de soufflage d'air chaud comporte des moyens de chauffage de l'air à une température comprise entre 20° et 90° C environ ;
- l'enceinte comporte un revêtement interne calorifuge ;
- l'installation comporte un dispositif d'extraction de l'atmosphère interne de l'enceinte chargée en ozone ;
- une bouche d'aspiration d'aird'alimentation du dispositif de soufflage est reliée au bout froid d'un échangeur de chaleur, et une bouche d'extraction de l'atmosphère interne de l'enceinte est reliée au bout chaud de cet échangeur, de manière à transférer les calories de l'atmosphère chaude de l'enceinte à l'air froid d'alimentation du dispositif de soufflage ;
- l'activité de la source de rayonnements est inférieure à 3,7 10¹⁵ Bq (100 KCi) et comporte de préférence du cobalt 60.

Un exemple de réalisation de l'invention sera décrit ci-dessous en se référant aux dessins annexés dans lesquels :
- la figure 1 est une vue en coupe, en élévation, d'une installation de traitement selon l'invention, le tiroir de l'enceinte de traitement étant en position ouverte ;
- la figure 2 est une vue en perspective de l'installation de la figure 1, l'enceinte de traitement étant représentée en traits mixtes ;
- la figure 3 est une vue en coupe suivant la ligne 3-3 de la figure 1 ;
- la figure 4 est une vue similaire à la figure 3 dans laquelle le tiroir de l'enceinte de traitement est en position fermée.

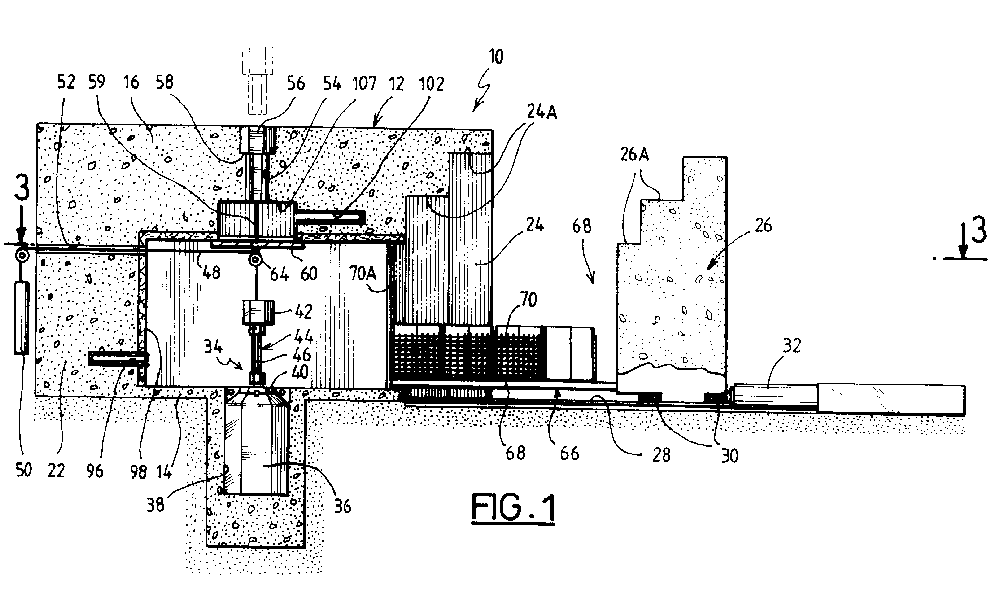

Sur les figures 1 à 4, on a représenté une installation de traitement de déchets hospitaliers selon l'invention, désignée par la référence générale 10. De préférence, l'installation 10 est disposée sur le site même d'un hôpital.

Les déchets sont habituellement contaminés par des microorganismes radiosensibles et/ou thermosensibles, tels que des gram+, des gram-, des myco- bactéries, des levures, des champignons, des virus ou des protozoaires.

L'installation 10 comprend une enceinte de décontamination 12, étanche aux rayonnements, de forme générale parallélépipédique, délimitée par une paroi inférieure formant sol 14, une paroi supérieure formant toit 16, et deux parois latérales opposées 18,20 adjacentes à une paroi latérale de fond 22.

Le côté de l'enceinte opposé à la paroi de fond 22 comporte une ouverture 24 obturable par une porte 26, montée coulissante sur des rails 28 horizontaux, parallèles à l'axe X de l'ouverture 24, par l'intermédiaire de moyens de roulement de type connu, par exemple des chenilles à rouleaux 30.

La porte 26 est déplacée au moyen d'un vérin hydraulique 32, par exemple télescopique.

De préférence, les parois 16-22 de l'enceinte ainsi que la porte 26 sont fabriquées en béton ordinaire, de densité 2,35, ou en béton baryté, de densité 3,10.

Les épaisseurs des parois 16-22 et de la porte 26 sont adaptées de manière à confiner les rayonnements internes à l'enceinte 12, selon les normes en vigueur. L'étanchéité aux rayonnements entre l'ouverture 24 et la porte 26 est assurée par des décrochements 24A,26A ménagés dans leurs bords de jonction.

Dans l'exemple décrit, le volume interne de l'enceinte 12 est d'environ 17m³.

Comme on peut le voir en particulier sur la figure 1, l'installation 10 comporte un ensemble 34 émetteur de rayonnements ionisants, de type connu, par exemple décrit dans FR-A-2 590 717.

Cet ensemble 34 comporte un conteneur 36, de préférence en plomb gainé d'acier inoxydable, destiné à être logé dans un puits 38 ménagé sensiblement au centre de l'enceinte 12 dans le sol 14 de celle-ci.

L'extrémité supérieure du conteneur 36 comporte une ouverture 40 obturable par un bouchon 42.

Une source radioactive 44, comportant de façon classique des barreaux en matériau ionisant, est fixée sur la face inférieure du bouchon 42. De préférence, les barreaux 46 comprennent du cobalt 60 et l'activité de la source 44 est inférieure à 3,7 10¹⁵ Bq (100 KCi). En variante la source radioactive 44 peut comporter du césium 137.

La source radioactive 44 est déplaçable entre une position sortie de traitement, telle que représentée à la figure 1, et une position escamotée de stockage à l'intérieur du conteneur 36, au moyen d'un câble de manutention 48 relié, par une première de ses extrémités, à la face supérieure du bouchon 42, et par une seconde extrémité, à un vérin de manoeuvre 50, disposé à l'extérieur de l'enceinte 12.

En variante, le vérin de manoeuvre 50 peut être remplacé par un dispositif à contrepoids de type connu. Par ailleurs, le câble de manutention 48 peut être doublé ou peut être remplacé par une chaîne.

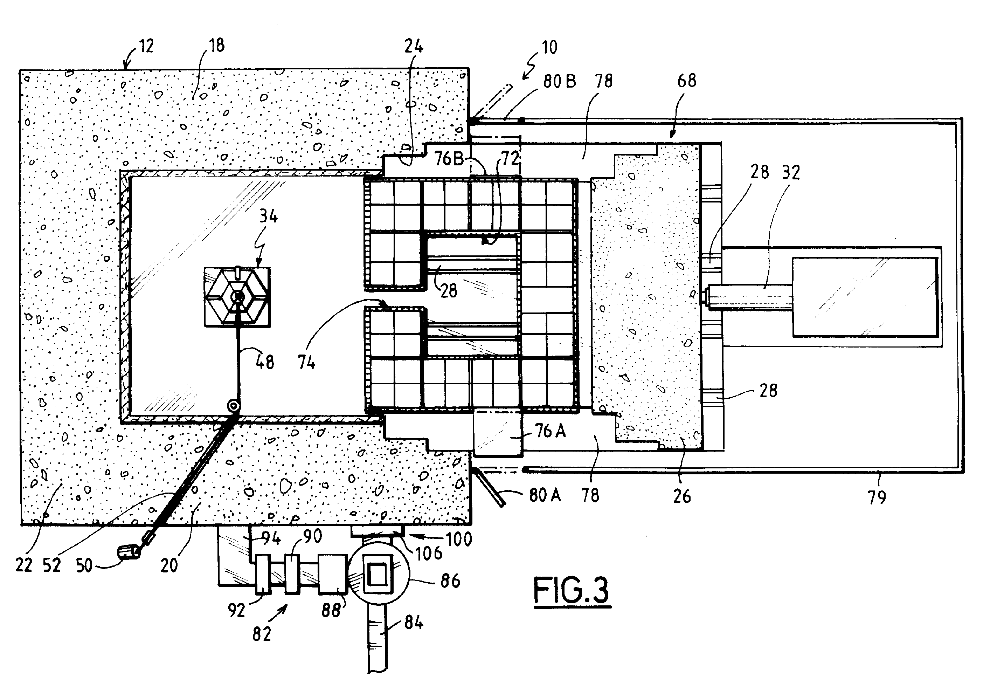

En se référant à la figure 3, on voit que le câble de manutention 48, guidé de façon classique par les poulies de renvoi, s'étend à travers un orifice rectiligne horizontal 52, formant fourreau, ménagé dans la paroi latérale 20 de l'enceinte. Ce fourreau 52 s'étend en biais par rapport aux faces de la paroi 20 de manière à éviter les fuites de rayonnements vers l'extérieur de l'enceinte 12.

On notera que, sur la figure 1, le vérin de manoeuvre 50 et le fourreau 52 du câble de manutention 48 sont représentés dans le plan de coupe de la figure, pour des raisons de clarté.

En se référant à nouveau à la figure 1, on voit que le toit 16 de l'enceinte comporte un orifice vertical 54, aligné avec le puits 38, formant un passage pour le câble d'un treuil (non représenté sur la figure) destiné à la manutention du conteneur 36.

Cet orifice de passage 54 est destiné à être obturé par un bouchon amovible 56, de préférence en béton, déplaçable de façon classique par treuillage. Sur la figure 1, le bouchon 56 est représenté en traits pleins, en position d'obturation, et en traits mixtes, en position sortie.

Le positionnement axial du bouchon 56 dans l'orifice 54 est réalisé par coopérât ion d'épaulements d'appui complémentaires 58 ménagés dans leurs contours respectifs.

Le bouchon 56 est retenu en position d'obturation par un système mécanique 59 reliant son extrémité inférieure à une plaque ajourée de sécurité 60, interne à l'enceinte 12. Cette plaque 60 est fixée à la face interne du toit 16 de manière à fermer l'extrémité de l'orifice de passage 54 débouchant dans l'enceinte 12.

Par ailleurs, la plaque 60 forme un support pour une poulie de renvoi 64 du câble de manutention 48 de la source de rayonnements 44.

Les liaisons mécaniques entre le bouchons 56 du toit et la plaque de sécurité 60, et entre cette plaque 60 et le câble de manutention 48, constituent des sécurités permettant d'éviter un démontage intempestif de ce bouchon 56.

En effet, le démontage du bouchon 56 est conditionné par les étapes préalables de démontage de la plaque de sécurité 60 et de découplage de cette plaque 60 et du bouchon 56.

De plus, le démontage de la plaque de sécurité 60 est conditionné par les étapes préalables d'obturation du conteneur 36 par le bouchon 42 portant la source de rayonnements 44, et de découplage de ce bouchon 42 et du câble de manutention 48.

De cette façon, le démontage du bouchon 56 du toit ne peut pas se faire accidentellement en cours de traitement des déchets, ou lorsque la source de rayonnements 44 est en position sortie.

Les déchets à traiter sont disposés dans un réceptacle 66 de chargement de l'enceinte, selon un procédé qui sera décrit ultérieurement.

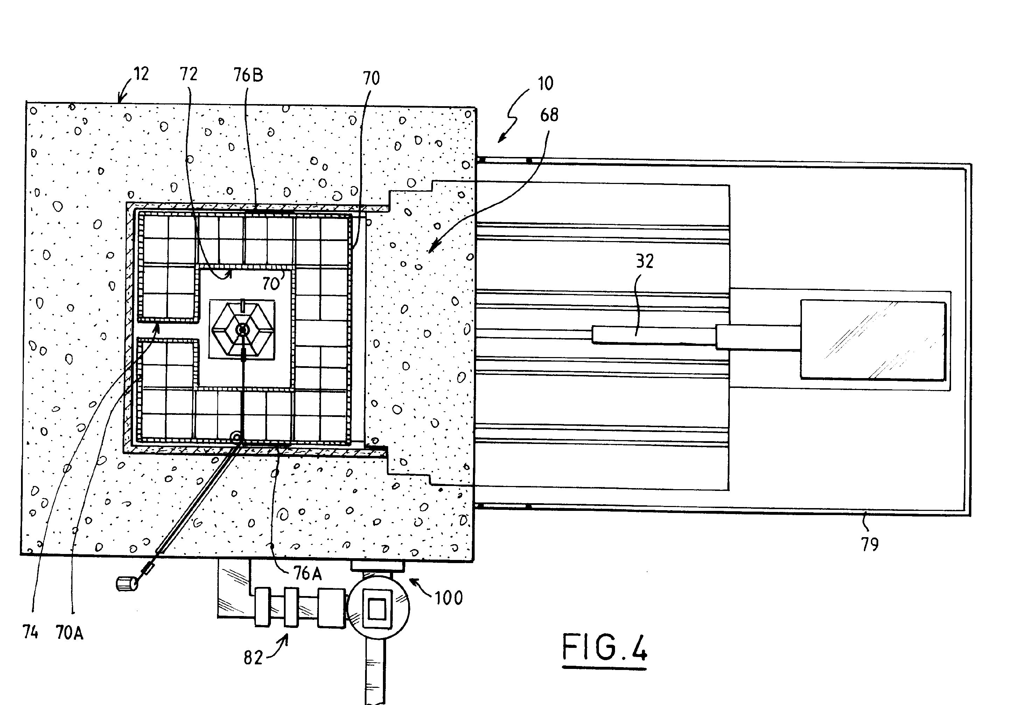

Ce réceptacle 66 est solidaire de la porte 26 de manière que l'ensemble porte 26-réceptacle 66 forme un tiroir 68 coulissant entre une position ouverte de chargement ou de déchargement des déchets, telle que représentée aux figures 1 à 3, et une position fermée de traitement de déchets, telle que représentée à la figure 4.

En position fermée, la porte 26 du tiroir interdit l'accès à l'enceinte pendant le traitement. De cette façon, la porte 26 du tiroir assure la sécurité d'accès à l'enceinte de traitement.

Le réceptacle 66, de forme générale parallélépipédique, est délimité par une paroi inférieure 68, formant plateforme de support des déchets, et des parois latérales 70,70A grillagées.

Dans ce qui suit, on qualifiera de proximal ou distal un élément respectivement proche ou éloigné de la porte 26.

La plateforme 68 est fixée en porte-à-faux, par son extrémité proximale, sur la face interne de la porte 26. Eventuellement, l'extrémité distale de la plateforme 68 peut comporter des roues d'appui destinées à être en contact avec le sol 14 de l'enceinte 12.

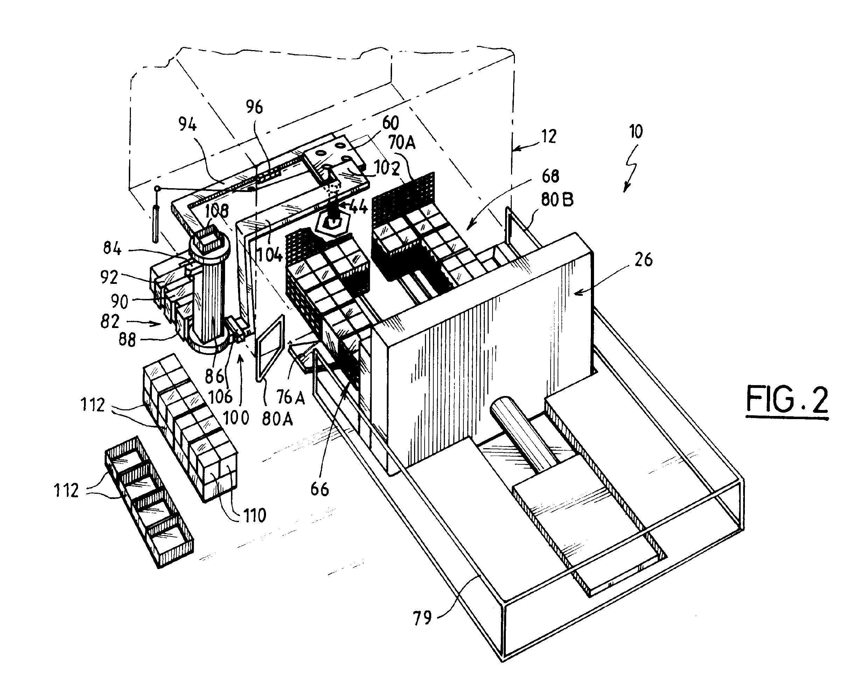

En se référant aux figures 1 et 2, on voit que la paroi latérale 70A, délimitant l'extrémité distale du réceptacle 66, s'étend sur une plus grande hauteur que les autres parois latérales 70, de manière à former une barrière empêchant l'accès d'un opérateur à l'intérieur de l'enceinte 12 lorsque le tiroir 68 est en position ouverte.

En se référant aux figures 2 à 4, on voit que le réceptacle 66 comporte un évidement central 72 formant logement pour la source de rayonnements 44 en position sortie, de manière que les déchets à traiter soient disposés autour de cette source 44.

Un évidement longitudinal 74, s'étendant entre l'extrémité distale et l'évidement central 72 du réceptacle 66, forme un passage pour le brin vertical du câble 48 relié à la source de rayonnements 44, lors des déplacements du tiroir 68.

Des portillons 76A,76B de chargement ou de déchargement du réceptacle 66 sont disposés symétriquement sur des côtés opposés de ce réceptacle en étant rabattables entre une position verticale de fermeture et une position horizontale d'ouverture. En position d'ouverture, les portillons 76A,76B enjambent un décrochement 78 du sol, délimitant un couloir de coulissement de la porte 26, de manière à former des passerelles d'accès au réceptacle 66.

La zone, adjacente à l'enceinte 12, dans laquelle s'effectue la course du tiroir 68, est clôturée par une barrière de sécurité 79. Cette barrière 79 comporte deux portes 80A,80B d'accès à la zone clôturée, disposées de manière à être sensiblement alignées avec les portillons 76A,76B du réceptacle 66 lorsque le tiroir 68 est en position ouverte.

L'installation 10 comporte de plus des moyens de chauffage des déchets à traiter qui seront décrits ci-dessous.

Ces moyens de chauffage comprennent un dispositif 82 de soufflage d'air chaud dans l'enceinte 12 alimenté par de l'air extérieur à l'enceinte.

Ce dispositif 82 comporte un conduit 84 d'aspiration d'air extérieur raccordé au bout froid d'un échangeur de chaleur 86, externe à l'enceinte 12. En sortie de cet échangeur 86, l'air préchauffé circule dans un circuit externe à l'enceinte comprenant, en série, un filtre 88, un ventilateur de soufflage 90 et un caisson de chauffage 92. Un conduit 94, encastré dans les parois de l'enceinte 12 relie la sortie du caisson de chauffage 92 à deux bouches de soufflage 96 communiquant avec le volume interne de l'enceinte 12, une seule de ces bouches étant représentée aux figures 1 et 2.

De préférence, les bouches de soufflage 96 sont disposées dans la partie basse de l'enceinte 12 et sont fermées par des grilles en acier inoxydable.

L'échangeur de chaleur 86 et le caisson de chauffage 92 sont adaptés pour permettre le chauffage de l'air à une température comprise entre 20 et 90°C environ.

De préférence, l'enceinte 12 comporte un revêtement interne 98 calorifuge, par exemple en laine de verre.

Sous l'effet des rayonnements ionisants, l'oxygène de l'air contenu dans l'enceinte 12 produit de l'ozone. Afin que la concentration en ozone dans l'atmosphère interne de l'enceinte 12 demeure acceptable, à tout moment, l'installation 10 comporte un dispositif 100 d'extraction de l'air ozoné.

En se référant aux figures 1 et 2, on voit que le dispositif 100 comporte une bouche d'extraction 102, communiquant avec le volume interne de l'enceinte 12, reliée par un conduit 104, encastré dans les parois de l'enceinte 12, à un ventilateur d'extraction 106, externe à l'enceinte 12.

De préférence, la bouche d'extraction 102 est disposée dans une partie haute de l'enceinte 12. Cette bouche 102 communique par exemple avec un élargissement 107 de l'orifice 54 du toit s'étendant entre l'extrémité inférieure du bouchon 56 et la plaque ajourée 60 (voir figure 1).

Le débit d'air du ventilateur d'extraction 106 est adapté pour que la concentration d'ozone à l'intérieur de l'enceinte 12 et dans l'effluent rejeté à l'extérieur de l'enceinte respecte les normes en vigueur. Par ailleurs, le débit d'air du ventilateur de soufflage 90 est adapté au débit d'air du ventilateur d'extraction 106 de manière à maintenir l'atmosphère interne de l'enceinte 12 en dépression par rapport à l'extérieur, pendant le traitement des déchets.

La sortie du ventilateur d'extraction 106 est raccordée au bout chaud de l'échangeur de chaleur 86 de manière à transférer les calories de l'air chaud ozoné, extrait de l'enceinte 12, à l'air froid d'alimentation du dispositif de soufflage 82.

En sortie de l'échangeur 86, l'air extrait de l'enceinte 12, comportant une faible concentration en ozone, conforme aux normes habituelles, est évacué à l'air libre par un conduit 108.

De préférence, les conduits 84,104 encastrés dans les parois de l'enceinte 12 sont fabriqués en acier inoxydable.

On décrira ci-dessous un procédé de traitement des déchets hospitaliers mettant en oeuvre l'installation 10 décrite ci-dessus.

Les déchets hospitaliers sont collectés et stockés habituellement dans des conteneurs 110 en carton de dimensions normalisées, chaque conteneur 110 ayant un volume d'environ 50 litres.

En vue du traitement des déchets, ces conteneurs 110 en carton sont regroupés par quatre dans des conteneurs 112 métalliques, fabriqués par exemple en aluminium ou en acier inoxydable (voir figure 2).

Dans l'exemple décrit, le réceptacle 66 peut contenir douze conteneurs 112 métalliques, ce qui correspond à 2,4 m³ de déchets à traiter.

Pour éviter des erreurs de manipulation des déchets, le chargement et le déchargement des déchets sont effectués par des côtés opposés du tiroir 68.

Sur la figure 3, les portillons 76A,76B du réceptacle 66 et les portes 80A,80B de la barrière 79 sont représentés en traits pleins, dans leurs positions adaptées au chargement du tiroir 68, et en traits mixtes, dans leurs positions adaptées au déchargement de ce tiroir 68.

Initialement, le tiroir 68 est en position ouverte.

On ouvre la porte 80A et le portillon 76A correspondant, affectés au chargement des déchets, puis on met en place les conteneurs métalliques 112 dans le réceptacle 66.

On referme le portillon 76A, la porte 80A et le tiroir 68.

On met en route le ventilateur d'extraction 106.

On lève la source de rayonnements 44 en position sortie de traitement de manière à irradier les microorganismes contaminant les déchets.

Pendant l'irradiation, on commande le ventilateur de soufflage 90 et le caisson de chauffage 92 de manière à effectuer un cycle de chauffage des déchets comprenant une période de chauffage à une température comprise entre environ 40 et 80°.

Les déchets sont irradiés pendant un temps tel que la dose de rayonnements absorbée par les déchets, en fin de traitement, est inférieure ou égale à 10 kGy. Dans l'exemple décrit, le temps d'irradiation des déchets est inférieur ou égal à 22 H.

Après décontamination des déchets, le chauffage est arrêté, la source de rayonnements est descendue en position de stockage dans le conteneur 36, et on arrête le ventilateur de soufflage 90 puis le ventilateur d'extraction 106.

Le déchargement des déchets décontaminés se fait après ouverture du tiroir 68, en accédant au réceptacle 66 par la porte 80B et le portillon 76B affectés au déchargement des déchets.

De préférence, l'enchaînement des étapes successives de chargement, de traitement, et de déchargement des déchets est contrôlé par des sécurités en cascades et est géré, par exemple, par un automate programmable.

La période du radioélément cobalt 60 est d'environ 5 ans. Ce radioélément perd, chaque année, 12% de son activité et par conséquent de sa capacité de traitement.

Lorsque l'activité de la source de rayonnements 44 est devenue trop faible, il est possible d'effectuer un échange standard de l'ensemble émetteur de rayonnements 34 de la façon suivante.

Initialement, on positionne la source de rayonnements 44 à l'intérieur du conteneur 36 par fermeture du bouchon 42.

On ouvre le tiroir 38, on démonte la paroi distale 70A du réceptacle 66.

On fixe le bouchon 42 sur le conteneur 36.

Ensuite, on détache le câble de manutention 48 du bouchon 42, on démonte la plaque de sécurité 60, on détache le bouchon 56 du toit de la plaque 60, et on extrait ce bouchon 56 de l'orifice 54.

Puis, on passe à travers l'orifice 54 le câble d'un treuil de levage, de type connu, on fixe l'extrémité de ce câble au conteneur 36, et on soulève le conteneur 36 de manière à l'extraire du puits 38.

On positionne la plateforme 68 du réceptacle 66 sous le conteneur 36, par déplacement du tiroir 68 vers l'intérieur de l'enceinte.

Ensuite, on pose le conteneur 36 sur la plate-forme 68, on le décroche du câble de levage et on l'extrait de l'enceinte par déplacement du tiroir 68 vers l'extérieur de cette enceinte.

Le conteneur 36 peut alors être manutentionné avec des moyens de type connu.

La mise en place d'un conteneur de remplacement se fait par des opérations inverses à celles décrites ci-dessus.

L'invention ne se limite pas au mode de réalisation décrit et illustré sur les figures.

En particulier, les moyens de chauffage à soufflage d'air chaud peuvent être remplacés par d'autres moyens de chauffage classiques.

L'invention comporte de nombreux avantages.

Elle permet de combiner l'effet microbiologique de la chaleur et des rayonnements en synergie et complémentarité, certains microorganismes contaminant les produits traités étant plus thermosensibles que radiosensibles et vice-versa.

La combinaison de la chaleur et des rayonnements permet de diminuer très notablement les doses de rayonnements nécessaires à la stérilisation des déchets hospitaliers par rapport à une stérilisation classique effectuée par irradiation, à température ambiante. Par conséquent, une source de rayonnements d'activité inférieure à 100 kCi est suffisante pour traiter les déchets.

Le traitement des déchets peut être effectué sur les lieux même de production de ces déchets, c'est-à-dire sur le site de l'hôpital, en évitant ainsi tout risque lié au transport de produits contaminés.

L'installation selon l'invention permet de traiter des déchets préalablement conditionnés dans des sacs, sans avoir à les déchiqueter.

Après décontamination, les déchets hospitaliers peuvent être facilement transportés et traités au même titre que des déchets ménagers.

L'installation de traitement des déchets selon l'invention est compacte, d'exploitation simple et fiable car les déchets sont traités par simple chargement dans une enceinte de traitement, sans déplacement de ces déchets au cours du traitement. Ceci permet notamment d'éviter les inconvénients liés aux risques de pannes des moyens convoyeurs utilisés dans les installations classiques pour déplacer les déchets au cours de leur traitement.

L'installation de traitement décrite ci-dessus permet de traiter les déchets produits annuellement par un hôpital d'environ 1000 lits.

## Revendications

1. Installation de décontamination de produits contenant des microorganismes, notamment des déchets hospitaliers, du type comprenant une enceinte (12) de traitement des produits, délimitée par des parois (24-22,26) étanches aux rayonnements ionisants, et une source de rayonnements ionisants (44) montée à l'intérieur de l'enceinte (12) en étant déplaçable entre une position sortie de traitement des produits et une position escamotée de stockage, caractérisée en ce que l'enceinte (12) comporte un tiroir (68) de mise en place des produits à traiter monté coulissant, à travers une ouverture (24) de l'enceinte (12), entre une position ouverte de chargement ou de déchargement des produits et une position fermée de traitement des produits.

2. Installation selon la revendication 1, caractérisée en ce que le tiroir comporte une porte (26) d'obturation de l'ouverture de chargement (24), étanche aux rayonnements, et un réceptacle (66) de chargement des produits à traiter, solidaire de la face interne de la porte (26).

3. Installation selon la revendication 2, caractérisée en ce que l'extrémité distale du réceptacle (66) est délimitée par une paroi (70A) formant une barrière empêchant l'accès d'un opérateur à l'intérieur de l'enceinte (12) lorsque le tiroir (68) est en position ouverte.

4. Installation selon la revendication 2 ou 3, caractérisée en ce que le réceptacle (66) comporte un évidement central (72) formant logement pour la source de rayonnements (44) en position sortie de manière que les produits à traiter soient disposés autour de cette source (44).

5. Installation selon la revendication 4, caractérisée en ce que le réceptacle (66) comporte un évidement (74) longitudinal, s'étendant entre l'extrémité distale (70A) du réceptacle et l'évidement central (72), formant un passage pour un câble (48) de manutention de la source de rayonnements (44) lors du déplacement du tiroir (68).

6. Installation selon l'une quelconque des revendications 2 à 5, caractérisée en ce que le réceptacle (66) comporte au moins un portillon (76A,76B) pour le chargement ou le déchargement des produits à traiter.

7. Installation selon l'une quelconque des revendications 1 à 6, caractérisée en ce que la zone, adjacente à l'enceinte (12), dans laquelle s'effectue la course du tiroir (68), est clôturée par une barrière de sécurité (79).

8. Installation selon les revendications 6 et 7 prises ensemble, caractérisée en ce que la barrière de sécurité (79) comporte au moins une porte (80A,80B) d'accès à la zone clôturée, disposée de manière à être sensiblement en vis-à-vis avec le portillon (76A,76B) du réceptacle (66) lorsque le tiroir (68) est en position ouverte.

9. Installation selon l'une quelconque des revendications 1 à 8, caractérisée en ce que le dessus (16) de l'enceinte (12) comporte un orifice (54) de passage d'un organe de manutention d'un conteneur (36) de stockage de la source de rayonnements (44), l'orifice de passage (54) étant obturé par un bouchon amovible (56) destiné à être retenu en position d'obturation par accouplement avec un organe de sécurité (60), fixé à l'intérieur de l'enceinte (12), formant support pour une poulie (64) de renvoi d'un câble (48) de manutention de la source de rayonnements (44).

10. Installation selon la revendication 9, caractérisée en ce que l'organe de sécurité comporte une plaque (60) fermant l'extrémité de l'orifice de passage (54) débouchant dans l'enceinte (12).

11. Installation selon l'une quelconque des revendications 1 à 10, caractérisée en ce qu'elle comprend des moyens (82) de chauffage des produits disposés dans l'enceinte de traitement.

12. Installation selon la revendication 13, caractérisée en ce que les moyens de chauffage comprennent un dispositif (82) de soufflage d'air chaud dans l'enceinte (12).

13. Installation selon la revendication 14, caractérisée en ce que le dispositif (82) de soufflage d'air chaud comporte des moyens (86,92) de chauffage de l'air à une température comprise entre 20° et 90°C environ.

14. Installation selon l'une quelconque des revendications 1 à 13, caractérisée en ce que l'enceinte (12) comporte un revêtement interne calorifuge (98).

15. Installation selon l'une quelconque des revendications 1 à 14, caractérisée en ce qu'elle comporte un dispositif (100) d'extraction de l'atmosphère interne de l'enceinte chargée en ozone.

16. Installation selon les revendications 12 et 15 prises ensemble, caractérisée en ce qu'une bouche (96) d'aspiration d'air d'alimentation du dispositif de soufflage (82) est reliée au bout froid d'un échangeur de chaleur (86), et une bouche (102) d'extraction de l'atmosphère interne de l'enceinte (12) est reliée au bout chaud de cet échangeur (86), de manière à transférer les calories de l'atmosphère chaude de l'enceinte (12) à l'air froid d'alimentation du dispositif de soufflage (82).

17. Installation selon l'une quelconque des revendications 1 à 16, caractérisée en ce que l'activité de la source de rayonnements (44) est inférieure à 3,71015 Bq (100 KCi) et comporte de préférence du cobalt 60.
